# EUROPEAN PATENT APPLICATION

(11) **EP 4 023 180 A1**
(43) Date of publication of application: **06.07.2022**
(21) Application number: 20217723.4
(22) Date of filing: 29.12.2020
(51) Int. Cl.: A61B 18/24, A61B 18/14

(54) **MULTI-SOURCE TISSUE ABLATION SYSTEM FOR THE INTERNAL TREATMENT OF PARENCHYMAL ORGANS, HOLLOW ANATOMICAL CONDUITS OR BLOOD VESSELS**

(71) Applicant: GEM S.r.l., 55049 Viareggio (LU) (IT); MYRA Medical Sàrl, 1073 Savigny (CH)
(72) Inventor: BRANCHETTI, Lodovico, Northamptonshire, NN18 8GG (GB); DI CECIO, Mario, 25060 Marcheno (IT); PASQUINO, Enrico, 1073 Savigny (CH); DI MODUGNO, Nicola, 70037 Ruvo di Puglia (IT)
(74) Representative: Grosfillier, Philippe

(57) **Abstract**

Tissue ablation system for the internal treatment of parenchymal organs, hollow anatomical conduits or blood vessels (7); said system comprising an EM wave generator (1) and a catheter (8-14,16-40) with an active distal end; characterized by the fact that said generator (1) includes at least two EM wave outputs (3-6).

## Description

### Field of invention

The invention relates to the tissue ablation within parenchymal organs, hollow anatomical conduits or blood vessels. It more precisely relates to the use of different kinds of electromagnetic (EM) waves, essentially radiofrequency (RF), microwaves (MW) and laser (LS).

### Background

During the past two decades, imaging-guided tumor ablation (IGTA) that used either chemical or thermal energy has emerged as one of the most effective loco-regional treatment modalities for small malignant hepatic tumors. The first tumor ablation technique to be introduced to clinical practice was the percutaneous ethanol injection (PEI), which is a chemical ablation of hepatocellular carcinomas (HCCs). In the early 1990s, however, thermal ablation using radiofrequency (RF) was developed and proved its superiority to PEI in terms of better survival and local control of the disease in patients with early-stage nonsurgical HCCs. Thereafter, other types of IGTA techniques such as microwave ablation (MWA), cryoablation, laser ablation, irreversible electroporation and high-intensity focused ultrasound (US) were developed and adopted for the treatment of malignant liver tumors. Among them, radiofrequency ablation (RFA) has been the most widely used method of IGTA for small malignant hepatic tumors, particularly HCC and colorectal cancer liver metastasis (CRLM), due to its safety and effectiveness as well as a reasonably good clinical outcome. Currently, it is unknown whether novel technologies will expand the clinical role of image-guided ablation and improve long-term patient outcomes with respect to RFA.

RFA treatment of stenotic atherosclerotic plaques in arterial blood vessels (coronary or peripheral arteries) has been recently proposed to be investigated besides the conventional mechanical balloon angioplasty or drug eluting stenting or ballooning.

### Radiofrequency (RF)

Regarding RFA for the management of patients with HCCs, previous studies have reported that the overall survival after RFA for early-stage HCC was similar to that of surgical resection. Radiofrequency has been the most studied ablation modality in the treatment of colorectal liver metastases.

The wide range of local tumor progression (LTP) reported for the percutaneous approach (12%-48%), when compared to resection, limited its use to highly selected patients with small, well-positioned tumors or with liver recurrences after hepatectomy. Ablation with sufficient radiographic margins and histologically proven necrosis significantly lower LTP.

RFA uses an alternating electric current oscillating between 200 and 1200 kHz [15]. The radiofrequency electrode acts as a cathode. The ions of targeting tumors adjacent to the electrode tip vibrate rapidly in response to these alternating currents. This vibrating friction energy is transformed into heat, while energy deposition drops exponentially away from the tip. Tissue interaction with the temperature induced by radiofrequency is similar to that of laser. The revised 2017 thyroid RFA guidelines by Korean Society of Thyroid Radiology suggest several standard techniques. For benign thyroid nodules, perithyroidal lidocaine injection is recommended to control pain. The trans-isthmic approach and moving-shot technique are essential for thyroid lesions. This technique is useful to minimize complications and marginal nodule regrowth. Recently, a novel technique, named "vascular ablation technique" has been reported. Two different vascular ablation techniques are suggested: artery-first ablation and marginal venous ablation (venous staining). These techniques have the potential to enhance treatment efficacy and reduce the risk of regrowth. For recurrent thyroid cancers, guidelines recommend careful evaluation of critical structures before ablation, hydro-dissection to reduce the risk of thermal damage to surrounding critical structures, and the moving-shot technique.

Among possible complications, nerve damages are the most serious and feared one during RFA. Particularly, voice change induced by thermal damage of recurrent laryngeal nerve is the most common complication related to nerve damage. To lower the risk of damage, some technical measures have been suggested. Recently, cold 5% dextrose solution injection was introduced to treat nerve damage during ablation. If symptoms of nerve damage occur during RFA, such as voice change, palpitations, Horner syndrome, shoulder movement problems, or paresthesia, ablation should be immediately stopped and a cold 5% dextrose solution is injected directly into the space in which the nerves were located. In most patients, the cold fluids can treat effectively the thermal damage of nerves.

Most operators performing thyroid RFA use a thyroid- dedicated straight-type internally cooled electrode with short shaft (7 cm) and small diameter (18-19 gauge). In small recurrent thyroid cancers or parathyroid lesions, guidelines recommend 19-gauge electrode tip (i.e., 3.8 mm or 5 mm active tips). A recently introduced thyroid-dedicated bipolar electrode has been introduced for pregnant women and for patients carrying electrical devices (i.e., pacemaker).

Radiofrequency emission has been successfully tested in-vivo, in animal model, on atherosclerotic plaques induced in peripheral vessels. The effects of RFA on atherosclerotic plaques and the vessels structures has been studied and it can be stated that it doesn't induce any anatomical damages to the vessel tunicae. The action of RFA on the plaque is evident with decellularization and significant reduction of the neovessels in the tunica media and intima. The cell decellularization is mainly in charge of smooth muscle cells (SMC) responsible for the atherosclerotic plaque proliferation and the vessel restenosis after angioplasty. The inventive technology reported in this patent is also based on the application of RFA alone or associated with MW or LS together with stenting of blood vessels. It means that energy emission will be associated during vessel stenting or later on in case of restenosis.

### Microwave (MW)

Microwave (MW) ablation is gaining consensus in the ablation of liver tumors with the hope of capitalizing on its potential benefits over RF ablation, which were demonstrated mainly in animal studies. These include no need for grounding pads, less susceptibility to the heat sink phenomenon, larger ablation zones, shorter ablation times, and possibly better local tumor control. Previous clinical comparisons of MW ablation to RF ablation suggested an advantage for MW ablation with regard to local tumor control for CLM.

MW ablation proved to be resilient to the heat sink effect compared to RF ablation, offering good control for perivascular tumors. The LTP rates for the small number of CLMs treated are similar between RF ablation and MW ablation. The use of MW ablation can be preferred to RF ablation due to its improved ability to treat perivascular tumors, as well as its ability to achieve ablation in significantly reduced times and with no need for grounding pads.

### Laser (LS)

Laser (an acronym for 'light amplification by stimulated emission of radiation') is a highly coherent, collimated and monochromatic energy that can be precisely delivered into small targets from a primary source or through optical fibers that allows a great variability in length and shape of applicators. Common primary sources are laser diode or neodymium-yttrium aluminum garnet, which produce optical wavelengths of 820 nm or 1064 nm. When laser light hits the target, a local increase of temperature occurs, causing permanent damages such as coagulative necrosis (46-100 °C) and tissue carbonization/vaporization (100-110°C). The grade and rapidity of tissue damage depend on many factors, including the amount of energy released, the application time, the vascularization and the water content of the tissue. Laser ablation in the neck is usually performed under ultrasound (US) guidance, which allows a real-time monitoring of the procedure.

Before ablation, a comprehensive assessment of the target lesion to treat is performed with US or contrast-enhanced US (CEUS). The size and shape of the nodule, along with the spatial relations with adjacent organs needs to be carefully evaluated to avoid partial treatments or injury to surrounding organs. Local anesthesia is generally used, with or without conscious sedation depending on patient anxiety and operator preference. According to the size and shape of the nodule, one or more 21-gauge needles are inserted under real-time US guidance in the deepest portion of each nodule. Up to four needles can be inserted simultaneously. A distance of 1 cm should be ideally maintained between inserted needles. Then, a 300 mm diameter plane-cut quartz optical fiber is inserted and advanced up to the introducer needle tip. The introducer needles are then pulled back so as to expose the tip of each fiber at least 5 mm in direct contact with the target tissue. Common protocols are based on a mean power of 3-5 W, for a total energy delivery of 1200-1800 J for every illumination. After the first illumination, the fiber(s) can be withdrawn by 1/1.5 cm and a subsequent illumination can be performed (*pull-back* technique) until the whole target has been illuminated. Tissue ablation can be monitored in real time following the enlargement of iperechogenicity due to gas formation during treatment. For benign nodules, the aim of the treatment is to achieve a volume reduction of at least >50%, and a variable amount of viable nodular tissue is generally maintained in the periphery of the nodule, also in order to reduce the risk of possible complications. Conversely, for malignant nodules, complete tissue destruction needs to be achieved. Thus, laser ablation for malignant nodules requires a careful assessment of the surrounding structure and an idoneal localization of the nodule, ideally 5 mm far from the thyroid capsule. Hydro dissection can be performed to displace other structures close to the tumor. After the ablation, CEUS can be performed to better identify the actual extent of the ablation area, which can be generally overestimated by the gas formed during ablation; a further ablation can be performed during the same session in case CEUS demonstrate untreated areas. Laser ablation is generally performed as an outpatient procedure that does not require particular medication apart corticosteroids or analgesics in the rare case of post-ablation pain.

According to the state of the art, the most promising oncologic treatments are based on emission of three different energy sources, namely radiofrequency (RF), Microwave (MW) and Laser (LS). It appears clear, from the clinical data on comparative studies involving RF vs. MW or RF vs. LS, that each energy source has limits and advantages.

European patent application EP 3 355 821 A1 discloses several devices that each provide one of those three energy sources. Nowadays, three separate devices are therefore needed if the three energy sources are required.

Accordingly, there is a need to provide all three energy sources in a more convenient way.

### Summary of the invention

The present invention concerns a tissue ablation system for the internal treatment of parenchymal organs, hollow anatomical conduits or arterial/venous blood vessels; said system comprising an EM wave generator and a catheter with an active distal end. The system according to the invention is characterized by the fact that the generator includes at least two EM wave outputs.
The invention also includes a catheter that is adapted for this tissue ablation system.

In a preferred embodiment, the system according to the invention provides the three different energy sources, i.e., RF, MW and LS, separately or in combination, during the same procedure.

In particular the invention describes a generator of electromagnetic waves such as RF, MW and LS to which are connected needles/catheters for tissue ablation able to support the delivery of two associated energy sources. For example, a needle delivering RF and MW in alternate switch modality of pulses at millisecond intervals. Or needles able to deliver RF and LS or MW and LS energies with variable ablation field volumes and a cooling system with temperature controlled by thermocouples or thermistors.
The generator for the three energy sources is equipped with specific energy source generators and a dedicated software able to potentially emit all sources at the same time, preferably two associated energy sources any time depending upon the therapeutic indications decided by the operator. The generator and its software are designed to control the ablated tissue temperature and impedance during the procedure, depending on the selected energy sources, acting on the cooling system that can be based on liquid CO₂, refrigerated air or water.
The needles or catheters are in general small, to reduce the tissues' disruption, and the dimensions are depending on the organ to be treated by thermal ablation and the adopted cooling system. For example, needles could have a diameter of 16 G (Gauge) or smaller and the catheters a diameter of 6 F (French) or smaller. An additional feature of the ablation needles/catheters is comprising a mechanical biopsy sampler placed on the tip or replacing the tip that is also acting as electrode. This feature is allowing to harvest tissue samples for histologic assessments after an ablation procedure.
The use of a catheter is indicated for all tissue ablations in hollow organs such as, for example, brain circulatory vessels, heart vessels (coronary arteries), arterial or venous blood vessels such as the pulmonary veins (arrythmia treatment), kidney blood vessels, respiratory tree, liver biliary conduits, gastrointestinal tract, bladder/ureteral vessels or reproductive organ cavities.

### Detailed description of the invention

The invention will be better understood in the present chapter, with some non-limiting illustrated examples.

### Brief description of the figures

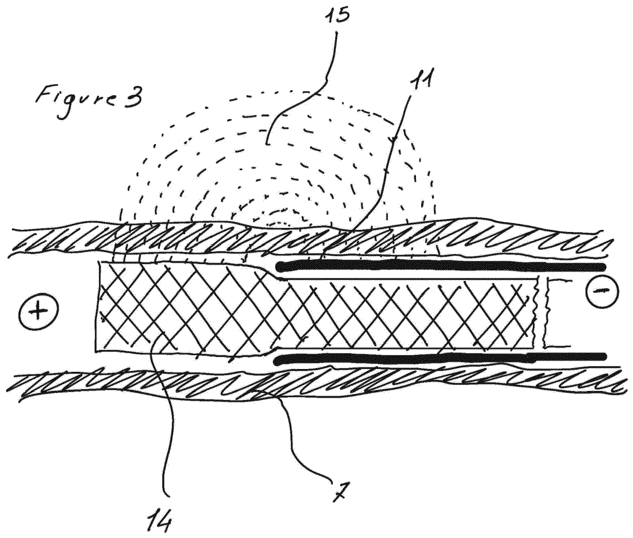

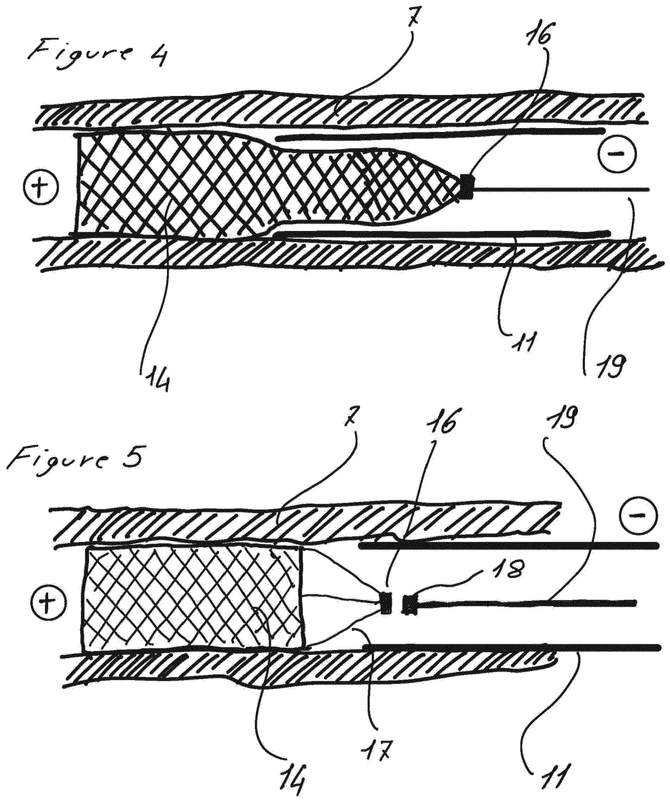

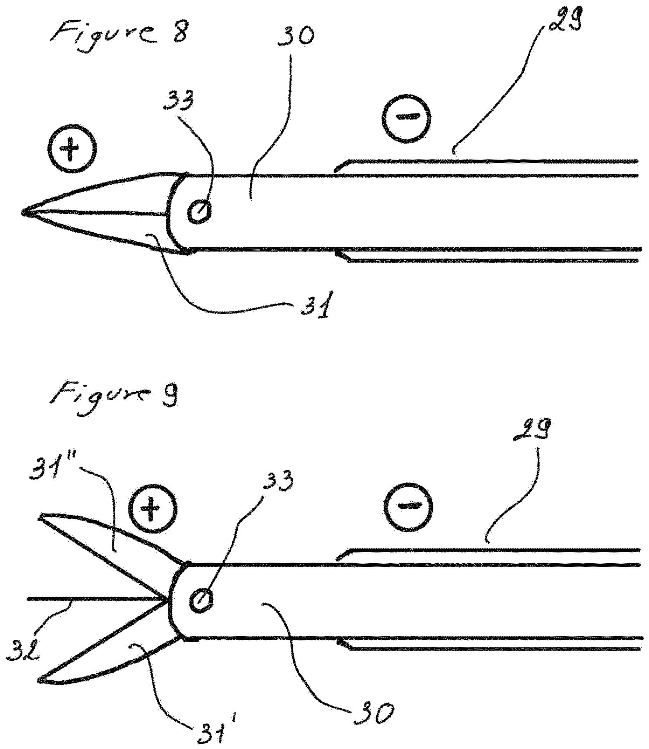

1. Generator of multisource energies;
2. Catheter with variable energy field given by an expandable metallic stent collapsed into an outer catheter;
3. Variable energy field catheter based on an outer catheter acting as cathode and an inner anode electrode realized with an expandable and recapturable stent. The exposure length of the inner stent anode determines the dimension of the energy field;
4. Variable energy field catheter similar to the embodiment represented in Figure 3. The inner stent anode is partially deployed and the extension of deployment is impacting on the generated energy field;
5. Same catheter device represented in Figure 4. The ablation procedure is completed, the stent left in place in the conduit and the outer catheter retracted. This maneuver allows the operator to redo the ablation procedure later on eventually repositioning the stent. The stent can be left in place at long-term or removed;
6. Multipolar needle/catheter for extended tissue ablations. The outer cathodic catheter is carved out exposing for a predetermined length the anodic inner catheter. The result of this multipolar design is that two energy fields are created for special tissue ablation needs;
7. Laser ablation catheter with variable energy field and associated cooling system. The picture shows the structural sections of the laser catheter;
8. Biopsy tweezer-ablation catheter, integrated with a variable field ablation needle/catheter, is able to conduct tissue ablation procedures associated with biopsy tissue sampling (before and/or after the procedure) and injecting chemotherapy drugs or other fluids useful for the success of the procedure;
9. Same catheter as described in Figure 9. In this Figure the catheter shows the biopsy jaws in open position and an internal needle aimed at injecting fluids;
10. RF and MW combined needle/catheter equipped with MW emission antenna, temperature control thermistor/thermocouple and cooling system.

### Numerical references used in the figures

1. Pulse generator for multisource energy delivery;
2. Screen reproducing ramp of impedance or temperature during an ablation procedure;
3. Radiofrequency (RF) connection plug;
4. Microwaves (MW) connection plug;
5. Laser (LS) beam connection plug;
6. RF&MW connection plug;
7. Blood vessel or anatomic conduit into which an ablation treatment is performed;
8. Expandable metal stent that fits the conduit wall. It is connected on both sides to the cathode (11,12);
9. Catheter's tip;
10. Inner catheter inserted into the outer catheter (11) acting as anode in its main portion and cathode (12) in its distal portion in continuity with the tip (9);
11. Outer catheter acting as cathode;
12. Distal portion of the inner catheter (10) negatively charged with its tip (9);
13. Inner lumen of the conduit into which biologic fluids (e.g., blood) are flowing through;
14. Inner positively charged electrode (anode) in shape of an expandable stent contained into the outer negatively charged electrode (cathode) in shape of flexible or rigid catheter (11) ;
15. Energy field created by the interaction between two electrodes;
16. Stent connector to the inner shaft connector (18);
17. Tethering cables connecting the stent (14) to the inner shaft (19) by means of stent connector (16) and shaft connector (18);
18. Shaft connector matching the stent connector (16);
19. Inner shaft connecting the stent;
20. Inner shaft anodic electrode;
21. Outer shaft cathodic electrode;
22. Laser optical fiber;
23. Outer catheter on which the balloon (24) is sealed-on;
24. Balloon made of a polymeric film;
25. Inlet lumens inside the thickness wall of the outer catheter (23) for cooling gas;
26. Outlet lumens inside the thickness wall of the outer catheter (23) for cooling gas;
27. Device tip;
28. Interspace (cooling space) between the balloon (24) and the optical laser fiber (22);
29. Outer cathodic catheter;
30. Inner anodic catheter;
31. Biopsy jaws in closed position (33' and 33" the jaws are open) the jaws are hollow inside in order to contain biopsy tissues;
32. Inner needle positioned inside the inner anodic catheter (32). It allows to inject fluids;
33. Hinge mechanism for opening/closure of the jaws (33' and 33");
34. Catheter tip;
35. Inner catheter anodic electrode;
36. Microwave antenna;
37. Thermistor or thermocouple. Cable connection 37';
38. Outer catheter cathodic electrode;
39. Cooling system lumens;
40. Lumen of the inner catheter anodic electrode (35).

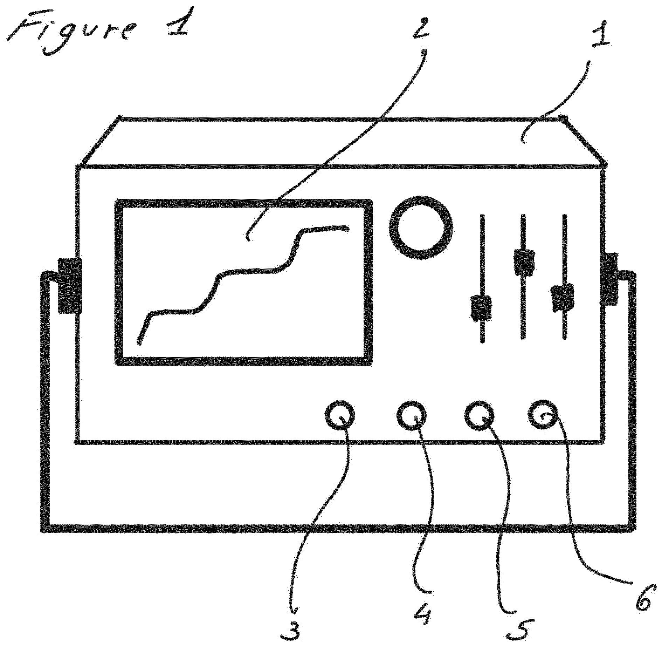

The pulse generator described in figure 1 is equipped with a monitor showing the ablation parameters and typically the temperature increase ramp or the impedance and a series of energy outputs such as an RF output (3), a MW output (4), a LS (5) and joint RF and MW output (6).

Several cursors will complete the generator with the function to regulate the different ablation functions.

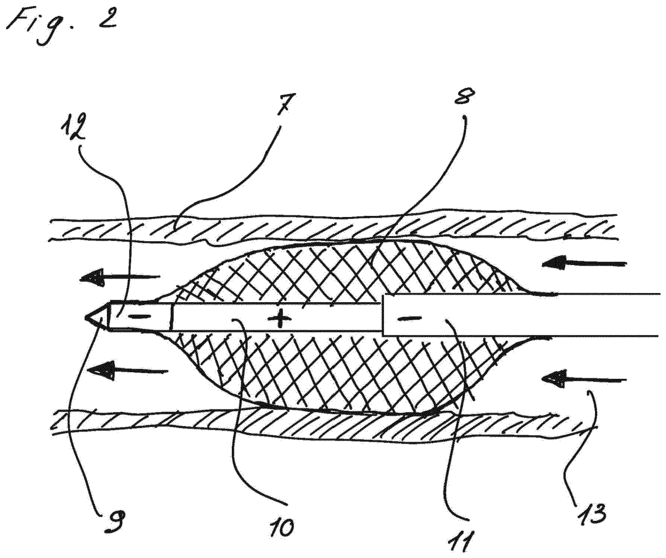

In figure 2 an RF ablation procedure in a hollow anatomical conduit (vessel, biliary duct, etc.) is illustrated. A metallic mesh (8) is expanded and fits the conduit wall (7) and allows any kind of body fluids to cross it preserving a flow circulation (13). The balloon-like metal mesh (8) is proximally anchored to a negatively charged external shaft (11) and distally anchored to a negatively charged internal shaft (12). The external cathode shaft (11) and the internal anode shaft (10,12) with its tip (9) are coaxial and telescopic. The internal shaft has two portions: one anodic (10) and one cathodic (12). The relative movement of the outer shaft (11) in respect to the internal shaft (10,12) determines the creation of a variable electrical field able to ablate tissue of the conduit's wall.

In figure 3 an example of a tissue ablation associated with a stenting procedure in a conduit (7) is shown. A metallic self-expandable stent positively charged (14) is contained in a hollow negatively charged shaft (11). The ablation field (15 must be considered symmetrical) generated is variable in dimension more or less depending on the exposure length of the stent (14).

Figure 4 and 5 represent two stages of stent deployment in a vessel or conduit (7). In this case the stent is intended to be left in place after the ablation procedure. The self-expandable stent, acting as anode (14), is fully contained in a hollow shaft catheter (11) acting as cathode. The stent (14) is retained, inside the shaft (11), by a wire (19) and connected to the wire through a quick connector (16). The stent's quick connector (16) is connected to the wire by means of a connector (18). The stent's connector is anchored to the stent with tethering wires or other similar embodiments. The stent's connectors (16) and the wire's connector (18) are designed to couple each other with different mechanical solutions, magnetic coupling or others. This inventive solution allows the stent to be captured later on, depending on the clinical follow-up, and the ablation procedure repeated, or the stent repositioned in a different location. In another embodiment the ablation procedure can be repeated, during clinical follow-up inserting inside the stent a cathode fitting the internal stent's wall that will be positively charged through a wire connection (16,17,18,19). Typically, an electrified ablation balloon, an electrified stent or other similar embodiments.

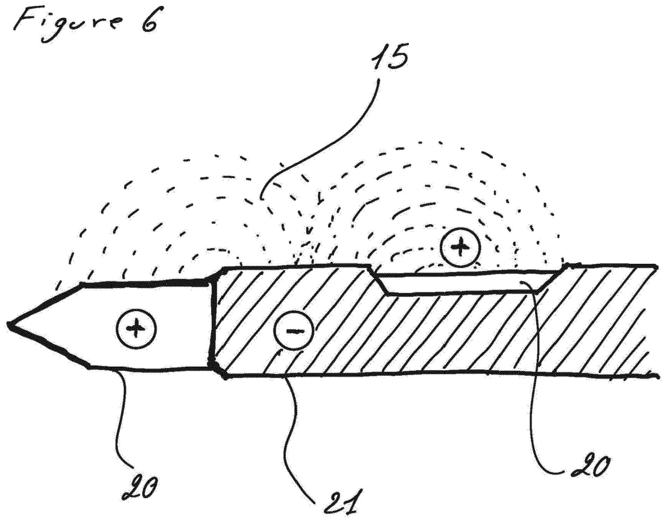

A telescopic needle/catheter emitting two ablation energy fields (15) in figure 6 is represented. The external catheter (21) acts as cathode while the internal one (20) is acting as anode. The two catheters (20,21) are moving relatively each other creating an ablation field. In the present embodiment the external catheter shaft (21) is scalloped for a certain length exposing the surface of the internal catheter shaft (20). This condition is creating a second ablation field (15). With this inventive solution two contiguous ablation fields (15) are generated allowing an extended ablation surface. Several variations can be envisaged for this ablation catheter including the number, the length and the shape of the scalloped ablation surfaces.

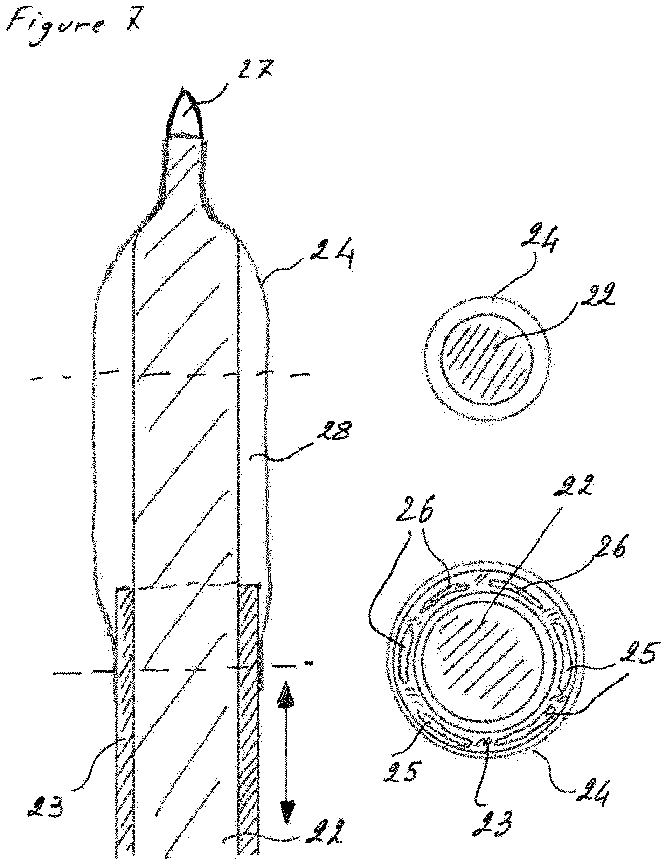

A LS tissue ablation application is described in figure 7. The laser optical fiber (22) acts as internal shaft of an external multilumen catheter (23). The two elements (22,23) are moving relatively and telescopically. In this embodiment the laser tissue ablation catheter is gas-cooled to mitigate the tissue carbonization a critical issue of the laser ablation procedures. The gas-cooling system is obtained creating an interspace or an isolated chamber (28) around the optical fiber (22). The interspace (28) is created by a thin polymeric film balloon (24) proximally anchored to the external catheter (23) and distally anchored to the optical fiber (22) and the tip (27). The gas-cooling system is maintained with CO₂ gas, or other gases/liquids, injected into the interspace through multilumen channels of the external shaft (23). The gas is injected through the inlet lumens (25) and it is expelled through the outlet lumens (26). The cooling of the optical fiber is obtained maintaining a positive pressure inside the interspace (28) by regulating the inlet and outlet gas pressure also in relationship to the telescopic extension of the outer catheter (23) and the optical fiber (22).

In figure 8 and 9 a telescopic ablation needle/catheter with biopsy and drug injection capability is described. The anode is an internal shaft catheter (30) inside an external shaft catheter (29) negatively charged. The two structures (29,30) are telescopic and the relative movement can determine a different dimension of the energy ablation filed.

The additional inventive embodiment of this ablation needle/catheter (30) is the possibility to harvest a biopsy samples from the ablated tissue and to inject solutions or drugs before, during and after the ablation procedure. In figure 8 the ablation device is represented closed while in figure 9 the device is represented with the biopsy jaws (31) in open position (31', 31") with the injection needle (32) extended and visible. The biopsy jaws (31', 31") are hinged (33).

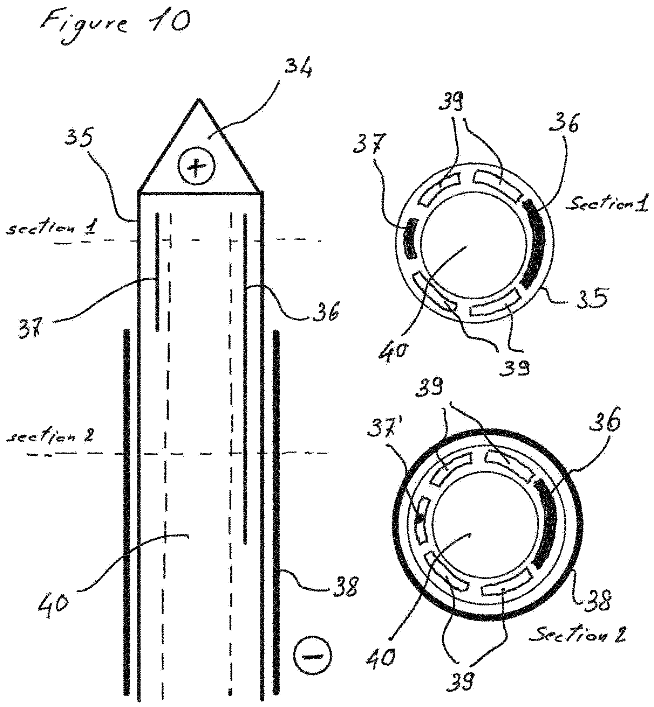

A hybrid RF and MW ablation needle/catheter is represented in figure 10. The needle/catheter is characterized by a negatively charged external shaft (38) with inside, moving telescopically, a positively charged internal shaft (35) ending with a tip (34). This ablation needle/catheter represented in this embodiment is able to perform an ablation procedure with only RF, only MW or in case alternatively RF and MW with a predefined and programmable time frame. This inventive solution could bypass the limitations of both ablation treatments providing an optimal ablation procedure. The MW antenna (36) is positioned inside the inner shaft (35) and the thermistor (37) measuring the temperature during the ablation procedure. The multilumen inner shaft (35) has additional inlet and outlet lumens (39) for the cooling of the needle/catheter with gases or liquids. The internal lumen (40) of the shaft (35) is deputed for injection of purging liquids or delivery drugs.

## Claims

1. Tissue ablation system for the internal treatment of parenchymal organs, hollow anatomical conduits or blood vessels (7); said system comprising an EM wave generator (1) and a catheter (8-14,16-40) with an active distal end; **characterized by** the fact that said generator (1) includes at least two EM wave outputs (3-6).

2. System according to claim 1 wherein one output (6) is a joint output that provides two types of EM waves, such as RF and MW.

3. System according to claim 1 or 2 comprising four outputs (3-6) that provide, separately or in combination, RF, MW and LS.

4. System according to any of claims 1 to 3 wherein said catheter comprises an internal shaft (10,20,22,30,35) and an external hollow shaft (11,21,23,29,38), both shafts being coaxial and movable relatively to each other.

5. System according to claim 4 wherein said catheter is a laser-based catheter, wherein the internal shaft is an optic fiber (22) and wherein the amplitude of the laser ablation field dimension is telescopically regulated by the relative movement of the external shaft (23).

6. System according to claim 5 wherein said laser furthermore comprises coolant outlets (26) that are adapted to provide a coolant around the free portion of the optical fiber (22).

7. System according to claim 4 wherein said catheter comprises an anodic portion and a cathodic portion.

8. System according to claim 7 wherein said anodic portion is located on the internal shaft and wherein said cathodic portion is located on the external shaft.

9. System according to claim 8 comprising a balloon-like metallic mesh (8) and wherein the internal shaft also comprises a cathodic portion (12), said mesh (8) being located around the catheter distal end in such a way as to conductively connect the cathodic portion of the external shaft (11) to the cathodic portion (12) of the internal shaft (10) .

10. System according to claim 8 wherein said internal shaft is essentially made of a wire (19) and a detachable stent (14) that is connected to the wire distal end (18), said stent (14) forming said anodic portion.

11. System according to claim 8 wherein the external shaft (21) comprises a notch (20) that exposes the internal shaft (20) with the external environment, generating thereby a second energy field (15).

12. System according to claim 8 comprising biopsy jaws (31) and/or a drug injection needle (32) that are/is linked to the internal shaft (30) distal end.

13. System according to claim 8 comprising a MF antenna (36) located within the internal shaft (35) and wherein said catheter is adapted to provide MF alone, RF alone or a combination of both.

14. Catheter for use with a tissue ablation system for the internal treatment of hollow organs or blood vessels (7), wherein said catheter is a laser-based catheter as defined in claim 5.

15. Catheter for use with a tissue ablation system for the internal treatment of hollow organs or blood vessels (7), wherein said catheter is a RF and/or a MW catheter as defined in any of claims 7 to 13.
